(19) Europäisches Patentamt
European Patent Office
Office européen des brevets

(11) EP 2 319 586 A1

(12) **EUROPÄISCHE PATENTANMELDUNG**

(43) Veröffentlichungstag:
**11.05.2011 Patentblatt 2011/19**

(21) Anmeldenummer: **10009667.6**

(22) Anmeldetag: **21.12.2005**

(51) Int Cl.:
*A61Q 1/02* (2006.01) *A61Q 5/00* (2006.01)
*A61Q 17/04* (2006.01) *A61Q 19/00* (2006.01)
*A61K 8/06* (2006.01) *A61K 8/22* (2006.01)

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.03.2005 DE 102005011459**

(62) Dokumentnummer(n) der früheren Anmeldung(en) nach Art. 76 EPÜ:
**05826355.9 / 1 858 593**

(71) Anmelder: **BEIERSDORF AG**
**20245 Hamburg (DE)**

(72) Erfinder:
- **Meiring, Uta**
  **20177 Hamburg (DE)**
- **Bleckmann, Andreas**
  **22926 Ahrensburg (DE)**
- **Treu, Jens**
  **22844 Nordestedt (DE)**
- **Blatt, Thomas**
  **22880 Wedel (DE)**
- **Teuber, Frank**
  **22846 Nordestedt (DE)**
- **Mummert, Christopher**
  **29553 Bienenbüttel (DE)**
- **Kolbe, Ludger**
  **21255 Dohren (DE)**
- **Kunert, Jutta**
  **22880 Wedel (DE)**
- **Rathsack, Jessica**
  **21614 Buxtehude (DE)**
- **Heinsohn, Guido**
  **25348 Glückstadt (DE)**

Bemerkungen:
Diese Anmeldung ist am 16-09-2010 als Teilanmeldung zu der unter INID-Code 62 erwähnten Anmeldung eingereicht worden.

(54) **Verwendung kosmetischer Zubereitungen mit einem Gehalt an nicht molekular gebundenem freien Sauerstoff**

(57) Kosmetische Zubereitung in Form einer selbstschäumenden, schaumförmigen, nachschäumenden oder schäumbaren O/W-Emulsion enthaltend 2,5 bis 25 Vol-% molekularen, nicht an Trägerstrukturen gebundenen Sauerstoff - bezogen auf das Gesamtvolumen der Zubereitung - in einem Packmittel bestehend aus einem Bag-on-Valve-System oder Bag-in-Can-System.

EP 2 319 586 A1

## Beschreibung

Die vorliegende Erfindung betrifft die Verwendung kosmetischer Zubereitungen mit einem kosmetisch wirksamen Gehalt an nicht molekular gebundenem freien Sauerstoff zur Verbesserung des Aussehens der Haut und/oder der Hautanhangsgebilde.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr.

Eine für den Verbraucher sehr wesentliche, dabei aber nur schwer quantitativ meßbare Eigenschaft kosmetischer Produkte ist ein bestimmtes belebendes und/oder glattes Hautgefühl sowie die Geschmeidigkeit der Haut nach seiner Anwendung.

Eine rosige Hautfarbe (wie sie beispielsweise durch den Aufenthalt an der frischen Luft erhältlich ist) wird von den Verbraucherinnen und Verbrauchern als gesund und dementsprechend wünschenswert angesehen, wohingegen ein blasser Teint in der Regel nicht so erwünscht ist. Aufgabe der vorliegenden Erfindung war es, hautpflegende Zubereitungen und Zubereitungen zur Pflege der auf natürliche Weise gealterten Haut zur Verfügung zu stellen, wobei die Zubereitungen das Körperäußere pflegen und verschönern sollten.

Es hat sich überraschenderweise herausgestellt und darin liegt die Lösung dieser Aufgaben, daß die Verwendung von kosmetischen Zubereitungen in Form von O/W-Emulsionen, welche 2,5 bis 25 Vol.-% freien Sauerstoff - bezogen auf das Gesamtvolumen der Zubereitung - enthalten, zur Verbesserung des Aussehens der Haut und/oder der Hautanhangsgebilde den Nachteilen das Standes der Technik abhilft.

Die erfindungsgemäße Verwendung der kosmetischen Zubereitungen mit einem kosmetisch wirksamen Gehalt an nicht molekular gebundenem freien Sauerstoff - z. B. in Form der genannten Beispiele - führt in überraschender Weise zu einer erheblichen Verbesserung des Aussehens der Haut und/oder der Haare und/oder der Nägel, insbesondere verbessert die erfindungsgemäße Verwendung

- ■ die Hautfarbe und den Teint,
- ■ die Hautgeschmeidigkeit sowie
- ■ die Elastizität von Haut und Haaren und verbessert daher Cellulite bzw. die sog. "Orangenhaut".

Bevorzugt enthalten die Zubereitungen im Sinne der vorliegenden Erfindung 5 bis 20 Vol.-%, besonders bevorzugt 7,5 bis 15 Vol.-% freien Sauerstoff.

Durch die erfindungsgemäße Verwendung wird überraschenderweise insbesondere der Glanz der Haut erhöht, wobei selbstverständlich kein Fettglanz auftritt, sondern die glänzende Haut vielmehr gesund aussieht und sich auch gesund anfühlt.

Es war ferner überraschend, daß durch erfindungsgemäße Verwendung die Mikrozirkulation der Haut verbessert wird, wodurch diese eine rosige und ultra-glatte Ausstrahlung bekommt. Die Verwendung im Sinne der vorliegenden Erfindung führt daher in nicht vorhersehbarer Weise zu einer Belebung des Teints sowie zu einer frischen, rosigen und damit schöneren und jüngeren bzw. jünger wirkenden Hautfarbe.

In diesem Sinne stellt die erfindungsgemäße Verwendung also sozusagen den extra Atemzug reinen Sauerstoffs für eine gesunde und glänzende Haut dar.

Unter "natürlicher Farbton" bzw. "Verbesserung der Hautfarbe " ist im Sinne der vorliegenden Erfindung zu verstehen, daß bei erfindungsgemäßer Verwendung die Haut besser durchblutet und dadurch rosiger wird.

Die Hautfarbe bzw. Durchblutung der Haut lässt sich über die Bestimmung von LAB-Werten mittels Spectro PEN sowie durch spektroskopische Verfahren (Reflexionsspektroskopie) erfassen. Beide Verfahren werden im folgenden stichpunktartig erläutert:

### (1) Hautfarbe bzw. -Durchblutung (Colorimetrie):

- Messinstrument: **Spectro Pen** (Dr. Lange, Düsseldorf, D)
- Richtlinie: Working Instruction 4 STU 309

- Kenngrößen: CIE L*a*b*-System: L*-Wert als Maß für die Helligkeit, a*-Wert als Maß für die Rötung sowie b*-Wert als Maß für die Gelb- bzw. Blaufärbung, ggf. Berechnung des ITA-Wertes als Maß für den Fototyp gemäß folgender Formel:

$$(\text{ITA-Wert} = (\text{ARCTAN}((L-50)/b))*180/\quad)$$

- Messungen je Areal: 1; Auswertung des Originalwertes
- Interpretation: Reduktion der L*- oder ITA-Werte als Zeichen einer dunkleren Haut; Erhöhung der a*-Werte als Zeichen einer röteren Haut

**(2) Diffuse Reflexion (Hautfarbe bzw. -durchblutung):**

- Messinstrument: **Spektrometer MCS 500** mit Quarzlichtleiter (Zeiss, Jena, D)
- Richtlinie: Working Instruction 4 STU 316
- Kenngrößen: Fläche der Hämoglobinbanden bzw. Fläche unter der Reflexionskurve im sichtbaren Spektralbereich (380 bis 780 nm)
- Messungen je Areal: 2x 20 Spektren, online-Averaging der Einzelspektren;
- Auswertung des resultierenden Originalwertes
- Interpretation: Erhöhung als Zeichen einer besser durchbluteten, röteren Haut

Es hat sich überraschend herausgestellt, daß die wesentliche Grundlage der vorliegenden Erfindung die gepulste kurzzeitige topische Applikation von molekularem Sauerstoff ist. Durch einen kurzzeitigen Puls (max. 1 Stunde) kann zwar keine längerfristige Behebung eines Sauerstoffmangels erreicht werden, so dass sich die vorliegende Erfindung grundlegend vom Stand der Technik unterscheidet. Eine kurzzeitige, puls- bzw. bolusartige Stimulation des intra- und extrazellulären Sauerstoffgehaltes hat aber für die Zellen der Haut insofern Signalfunktion, als darüber verschiedene Mechanismen angeregt werden, in deren Folge die Zellen insgesamt zellphysiologisch aktiviert werden, ohne dass dabei die Nachteile, die mit einer Langzeit-Sauerstoffanwendung einhergehen, auftreten würden. Ein dauerhafter Ausgleich von Sauerstoffmangelzuständen in der Haut soll erfindungsgemäß nicht erreicht werden. Es ist aber vorteilhaft im Sinne der vorliegenden Erfindung, die pulsartige Applikation von molekularem Sauerstoff regelmäßig - z. B. 2-3 mal täglich - zu wiederholen.

Ein besonderer Vorteil der vorliegenden Erfindung ist, dass der molekulare Sauerstoff nicht an Trägerstrukturen gebunden ist. Dies hat zur Folge, daß ein schnelles An- und Abfluten des Gases durch die verschiedenen Schichten der Haut möglich ist. Lange Verweildauern und die damit einhergehenden Risiken von oxidativen und degenerativen Vorgängen, die bereits nach wenigen Stunden in einer stark sauerstoffhaltigen Atmosphäre entstehen können und dadurch dem erfinderischen Gedanken zuwider laufen würden, sind damit ausgeschlossen. Die dieser Erfindung zugrundeliegenden Daten belegen eindeutig den Vorteil einer gepulsten Applikation mit reinem molekularem Sauerstoff gegenüber einer dauerhaften Sauerstoffzufuhr oder der Applikation über Trägermoleküle.

Es ist besonders vorteilhaft im Sinne der vorliegenden Erfindung, den Sauerstoff in Form von selbstschäumenden, schaumförmigen, nachschäumenden oder schäumbaren kosmetische O/W-Emulsionen auf die Haut aufzubringen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Derartige Zubereitungen können vorteilhaft in Form von Emulsionen vorliegen und enthalten im Sinne der vorliegenden Erfindung vorteilhaft ein Emulgatorsystem, welches aus

A. mindestens einem Emulgator A, gewählt aus der Gruppe der ganz-, teil- oder nicht neutralisierten, verzweigten und/oder unverzweigten, gesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und

C. mindestens einem Coemulgator C, gewählt aus der Gruppe der gesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium-und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Paimitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-25-Glyceryltrioleat, PEG-15-Glycerylricinoleat, PEG-20-Glycerylstearat, PEG-20-Glycerylisostearat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist vorteilhaft im Sinne der vorliegenden Erfindung, die Gesamtmenge der Emulgatoren A und B und des Coemulgators C aus dem Bereich von 2 bis 10 Gew.-%, vorteilhaft von 2,5 bis 7 Gew.-%, jeweils bezogen auf das Gesamtgewicht der Formulierung, zu wählen.

Ferner vorteilhaft im Sinne der vorliegenden Erfindung sind Zubereitungen, welche in Form von Emulsionen vorliegen und einen oder mehrere O/W-Emulgatoren mit einem HLB-Wert von mehr als 6 enthalten. Besonders vorteilhaft werden der oder die O/W-Emulgatoren aus der Gruppe der ethoxylierten Fettsäuren und/oder der ethoxylierten Fettalkohole gewählt. Ganz besonders vorteilhafte sind Ceteareth-n, Steareth-n, PEG-n-Stearate und Polysorbate n, wobei n Werte zwischen 20 und 100 (entsprechend 20 bis 100 Ethoxyeinheiten) annehmen kann. Weitere vorteilhafte O/W-Emulgatoren im Sinne der vorliegenden Erfindung sind Glycerylstearatcitrat, Polyglyceryl-3-methylglucosedistearat sowie PEG-40 Castor Oil ggf. in Verbindung mit Natriumcetearylsulfat und/oder Cetearylalkohol.

Vorteilhaft enthalten O/W-Emulsionen im Sinne der vorliegenden Erfindung neben einem oder mehreren O/W-Emulgatoren ferner Co-Emulgatoren gewählt aus der Gruppe Glycerylstearat, Fettsäuren und Fettalkohole. Es ist besonders vorteilhaft im Sinne der vorliegenden Erfindung, den oder die Coemulgatoren aus der Gruppe Behenylalkohol, Cetylalkohol, Cetearylalkohol, Stearylalkohol zu wählen.

Ferner vorteilhaft im Sinne der vorliegenden Erfindung können die selbstschäumenden, schaumförmigen, nachschäumenden oder schäumbaren kosmetischen Emulsionen Substanzen enthalten, die die Grenzfläche Gas-Emulsion stabilisieren. Solche Grenzflächenstabilisatoren (eine oder mehrere Verbindungen) können z. B. aus der Gruppe der Füllstoffe gewählt werden. Die Füllstoffe können ferner z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Polymethylmethacrylate, Methacrylat Copolymere, Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9). Besonders vorteilhaft ist Talkum. Ferner vorteilhaft können der oder die Grenzflächenstabilisatoren aus der Gruppe der rheologischen Additive gewählt werden. Vorteilhaft sind insbesondere Aluminium- und/oder Magnesiumsilikate.

Ebenfalls vorteilhaft können die Emulsionen im Sinne der vorliegenden Erfindung auch Hydrokolloide (auch als Verdicker oder Gelbildner bezeichnet) enthalten, die die Zubereitungen stabilisieren können.

Als erfindungsgemäß vorteilhafte Hydrokolloide werden Agar-Agar, Carrageen, Tragant, Gummi arabicum, Alginate, Pektine, Polyosen, Guar-Mehl, Johännisbrotbaumkernmehl, Stärke, Dextrine, Gelatine, Casein, Celluloseether, Hydroxyethyl- und -propyl-cellulosederivate, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind : Magnesium- und/oder Aluminiumsilikate sowie beispielsweise Methylcellulosen, als welche die Methylether der Cellulose bezeichnet werden. Sie zeichnen sich durch die folgende Strukturformel aus

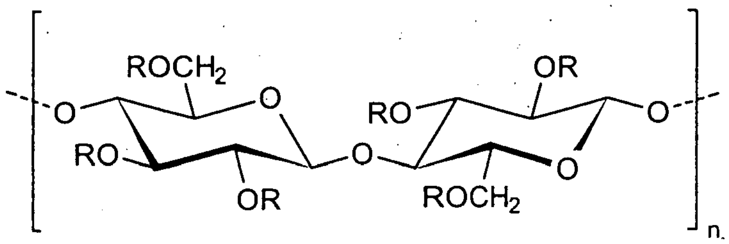

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Weitere vorteilhaft im Sinne der vorliegenden Erfindung einzusetzende Wirkstoffe sind solche, die den Zustand der Haut positiv beeinflussen, wie insbesondere Wirkstoffe zur positiven Beeinflussung der Altershaut, die die Entstehung von Falten oder auch bestehende Falten vermindern. Vorteilhaft sind insbesondere Biochinone, insbesondere Ubichinon (Q10) und Ubichinol, Folsäure und ihre Derivate (insbesondere Tetrahydrofolsäure und Dihydrofolsäure), Niacin und seine Derivate (insbesondere Niacinamid), Kreatin und Kreatinin, Carnitin, Biotin, Isoflavon, Cardiolipin, Liponsäure, Anti Freezing Proteine, Hopfen- und Hopfen-Malz-Extrakte. Auch Mittel, die die Restrukturierung des Bindegewebes fördern, wie natürliche und/oder synthetische Isofiavonoide sowie Isofiavonoidhaltige Pflanzenextrakte - wie z. B. Soja- und Klee-Extrakte - können in den erfindungsgemäßen Formulierungen sehr gut verwendet werden. Auch zeigt sich, daß sich die Formulierungen in besonderer Weise eignen, Wirkstoffe zur Unterstützung der Hautfunktionen bei trockener Haut (wie beispielsweise Vitamin C, Biotin, Carnitin, Propionsäure, Grüntee-Extrakte, Eucalyptusöl, Harnstoff und Mineralsalze (wie z. B. NaCl, Meeresmineralien) sowie Osmolyte (wie z. B. Inositol, Betain, quartäre Ammoniumverbindungen)) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süßholzes, Licochalcone, insbesondere Licochalcon A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxyaenase, aber auch des 5-Lipoxvgenase Inhibitor Proteins, FLAP. Insbesondere Kreatin und Kreatinin sind geeignete Wirkstoffe, um ein (Energie-) Depot anzulegen und/oder zu erneuern sowie die Reparatur (Repair) unterschiedlicher zellulärer Strukturen, insbesondere der DNA zu aktivieren. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen. Beispielhaft sei erwähnt Tyrosinsulfat, Dioic acid (8-Hexadecen-1,16-dicarbonsäure) sowie Liponsäure und Liponamid, verschiedene Extrakte des Süßholzes, Kojisäure, Hydrochinon, Arbutin, Alpha-Arbutin, Deoxyarbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin, Pyridoxamin, Niacinamid, Inhibitoren des Proteinase Activated Receptors 2 (PAR-2). Besonders bevorzugt sind ferner erfindungsgemäße Formulierungen, die weitere Wirkstoffe enthalten, die eine verstärkte oder schnellere Bräunung der Haut herbeiführen (Advanced Glycation Endproducts (AGE), Lipofuscine, NukleinsäureOligonukleotide, Purine und Pyrimidine, NO-freisetzende Substanzen), Tyrosin und seine Derivate (insbesondere N-Acetyl-Tyrosin), Phenylalanin und seine Derivate (insbesondere N-Acetyl-Phenylalanin), Aktivatoren des Proteinase Activated Receptors 2 (PAR-2), sei es mit oder ohne Einfluss von UV-Licht.

Erfindungsgemäße Rezepturen, welche z. B. bekannte Antifaltenwirkstoffe wie Flavonglycoside (insbesondere $\alpha$-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermai water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Die Ölphase der Zubereitungen im Sinne der vorliegenden Erfindung enthält vorteilhaft unpolare enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene und Isohexadecan. Unter den Polyolefinen sind Polydecene die bevorzugten Substanzen.

Es ist insbesondere vorteilhaft im Sinne der vorliegenden Erfindung, wenn der Gehalt an polaren Ölen mit einer Polarität von kleiner als 20 mN/m (Grenzflächenspannung gegen Wasser) kleiner als 5 Gew.-% - bezogen auf das Gesamtgewicht der Zubereitung - gewählt wird.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind synthetische Verbindungen, in denen Silicium-Atome über Sauerstoff-Atome ketten-und/oder netzartig verknüpft und die restlichen Valenzen des Siliciums durch KohlenwasserstoffReste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

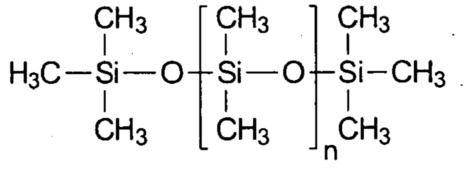

werden auch als Polydimethylsiloxan bzw. Dimethicon (INCI) bezeichnet. Dimethicone gibt es in verschiedenen Kettenlängen bzw. mit verschiedenen Molekulargewichten. Erfindungsgemäß vorteilhaft ist es, Cyclomethicon (Octamethylcyclotetrasiloxan) als Silikonöl zu verwenden. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenyisiioxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:

(a) Siloxanelastomere, welche die Einheiten $R_2SiO$ und $RSiO_{1,5}$ und/oder $R_3SiO_{0,5}$ und/oder $SiO_2$ enthalten, wobei die einzelnen Reste R jeweils unabhängig voneinander Wasserstoff, $C_{1-24}$-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten $R_2SiO$ zu $RSiO_{1,5}$ aus dem Bereich von 1 : 1 bis 30 : 1 gewählt wird;

(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind, wobei die verwendeten Mengenateile so gewählt werden, daß die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig,

gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |
| Tioveil AQ 10PG | Alumina / Silica | Solaveil / Uniquema |
| Eusolex T-aqua | Wasser/Alumina/Natriummeta-phosphat | Merck |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte UV-Filtersubstanzen im Sinne der vorliegenden Erfindung sind ferner:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxobicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

- Sulfonsäure-Derivate des 3-Benzylidencamphers, wie z. B. 4-(2-Oxo-3-bomylidenmethyl)benzolsulfonsäure, 2-Methyl-5-(2-oxo-3-bornylidenmethyl)sulfonsäure und deren Salze.
- Benzoxazol-Derivate, wie z. B. das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)-ester, 4-(Dimethylamino)benzoesäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Die Gesamtmenge der Filtersubstanzen wird aus dem Bereich von 0,1 bis 30 Gew.-%, vorzugsweise 0,5 bis 10 Gew.-%, insbesondere 1,0 bis 6,0 Gew.-% - jeweils bezogen auf das Gesamtgewicht der Zubereitungen - gewählt, um kosmetische Zubereitungen zur Verfügung zu stellen, die das Haar bzw. die Haut vor dem gesamten Bereich der ultravioletten Strahlung schützen.

Es ist besonders vorteilhaft im Sinne der vorliegenden Erfindung, die Sauerstoff enthaltenden Zubereitungen in Packmitteln bereit zu stellen, die einen minimalen Gasaustausch gewährleisten.

Derartige Packmittel sind z. B. aus der Zwei-Kammer-Technologie bekannt. Vorteilhaft sind beispielsweise die sog. Bag-on-Valve-Systeme, bestehend aus einem Ventil das hermetisch dicht mit einem Folienbeutel verbunden ist. Der Folienbeutel ist optimalerweise mehrlagig mit mindestens einer oder mehreren Sperrschichten. Das Beutelventil wird vor der Abfüllung hermetisch dicht mit einem Aerosolbehälter (Aluminium, Weißblech oder Kunststoff) verbunden.

Beim sog. Bag-in-Can-Systern befindet sich der Beutel in einem Aerosolbehälter und wird beim Verschließen mit dem Ventil und dem Aerosolbehälter verbunden.

Beschrieben sind derartige Packmittel in der DE-20301831, EP-972923, EP-1061006 oder EP-586295, die hiermit in den Offenbarungsgehalt der vorliegenden Erfindung eingeschlossen werden.

Der Behälter wird bevorzugt mit umweltfreundlichen Treibgasen, wie Stickstoff oder Edelgasen, beaufschlagt, wobei sich das Treibgas zwischen Beutel und verschlossenem Aerosolbehälter befindet, wohingegen sich das Kosmetikum im Beutel befindet.

Die Funktionsweise der Bag-in-Can-Systeme bzw. Bag-on-Valve-Systeme läßt sich wie folgt beschreiben: das Treibgas umgibt den mit dem Kosmetikum gefüllten Beutel. Wenn die Düse, der Sprüh- oder Applikationskopf des Systems, heruntergedrückt wird, sorgt der Druck auf den Beutel dafür, dass der Inhalt, je nach Durchmesser der Düse, in unterschiedlichen Mengen austritt. Dazu muss die im Beutel befindliche Zubereitung auf das Packmittel abgestimmt

werden, um bezüglich der Viskosität die richtige Ausbringung bei vorgegebenen Dosendruck zu erreichen.

Vorteil der Zwei-Kammer-Technologie (Bag-in-Can-Systeme, Bag-on-Valve-Systeme) ist, dass keine Verunreinigung und somit auch kein Luft in den unter Überdruck stehenden Beutel eindringen kann.

Der Spendekopf beinhaltet optimalerweise an der Austrittsöffnung einen Schließmechanismus, der eine mögliche Kontamination des Produktes zwischen Ventilöffnung und Austrittsöffnung reduziert .

Der selbstverschliessende Spendekopf kann z. B. wie folgt beschrieben ausgeführt werden: Durch Betätigung des Applikators wird ein Aerosolventil geöffnet, das Produkt strömt durch einen Kanal zur Austrittsöffnung. Beim Durchströmen des Kanals dehnt sich das elastische Element (z. B. aus einem thermoplastischen Elastomer (TPE), Polyethylen, Polypropylen, Polyoxymethylen (POM)), welches den Kanal bis zur Austrittsöffnung im unausgelösten Zustand verschlossen hält. Voraussetzung dafür ist ein höherer Doseninnendruck in Bezug zur Umgebungsatmosphäre. Beim Schliessen des Aerosolventils wird der Druck zwischen Aerosolventil und Austrittsöffnung bis zum kompletten Abdichten reduziert. Durch die Druckabnahme senkt sich das elastische Element in den produktführenden Kanal, und das verbleibende Füllgut wird bis zum vollständigen Verschliessen des Kanales appliziert.

Auf diese Weise wird eine Kontamination des verbleibenden Füllgutes im Spendekopf durch die Umgebungsatmosphäre bis zur nächsten Öffnung des Aerosolventiles verhindert.

Der selbstverschliessende Spendekopf kann ferner z. B. wie folgt ausgeführt werden:

> Durch Betätigung des Applikators wird ein Aerosolventil geöffnet, eine elastische Membran federt entgegengesetzt zum Produktstrom. Ein untrennbar mit der Membran verbundener Verschlusszapfen folgt dieser Bewegung, und die Austrittsöffnung / Ringschlitzdüse wird geöffnet.

Beim Schliessen des Aerosolventiles lässt der Produktstrom nach. Durch den Druckabfall bewegen sich Membran und Verschlusszapfen in ihre Ausgangsposition und die Austrittsöffnung / Ringschlitzdüse wird verschlossen.

Auch durch dieses Prinzip wird das Füllgut im Kopf optimalerweise geschützt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele: 1**

**Selbstschäumende, schaumförmige O/W-Lotions und Cremes**

| | **1** | **2** | **3** | **4** | **5** |
|---|---|---|---|---|---|
| Glycerinmonostearat SE | | | 1 | | |
| Stearinsäure | 2,00 | 3,0 | | | |
| Cetylstearylalkohol | 0,50 | | | | 2 |
| Myristylalcohol | 1,50 | | | 1 | |
| Cetylalcohol | | 2,00 | | 1 | |
| Polysorbate 60 | | | | | 1,5 |
| Glyceryl Stearate Citrate | | | | | 2,5 |
| PEG-40 Castor Oil, Sodium Cetearyl Sulfate, Cetearyl Alkohol | | | 3,5 | | |
| Steareth-21 | | | | 2,5 | |
| Steareth-2 | | | | 2,5 | |
| PEG-40 Stearat | | 5,50 | | | |
| PEG-100 Stearat | 3,00 | | | | |
| Cyclomethicon | | | 4 | 3 | |
| Squalan | | | 3 | | 2,5 |
| Trilaurin | | | | | |
| Avocadoöl | | | 0,5 | | |
| Dimethicon | | 4,50 | 2 | 2 | 1 |

(fortgesetzt)

| | **1** | **2** | **3** | **4** | **5** |
|---|---|---|---|---|---|
| Dicaprylyl Carbonate | | 2,00 | | | |
| Isohexadecan | | 1 3 | | | |
| Isoeikosan | | | | 4 | |
| Mineralöl / Paraffinöl | 4,00 | 4,50 | | 3 | 6 |
| Hydriertes Polyisobuten | 15,0 | | | | |
| Polydecen | | | 4 | 3 | 3 |
| Magnesium-Aluminium-Silikat | 0,20 | 0,05 | 0,1 | 0,1 | 0,2 |
| Hectorit | | 0,10 | | | |
| Quaternium-18-Hectorite | | | | 0,1 | 0,2 |
| Cellulosegummi | 0,2 | 0,3 | 0,1 | | |
| Polyacrylsäure | | 0,10 | 0,2 | | 0,2 |
| Polymethylmetacrylat | | | | | 2 |
| Alkyl Acrylates Cosspolymere | | | | | 0,2 |
| Hydroxypropyl Starch Phosphate und deren Ester | 0,25 | 0,5 | | | |
| Anorg. Pigmente (wie Boron Nitride, Zeolithe, Kaolin, Silicumoxid, Talkum, Silica Dimethyl Silylate etc.) | 1,25 | 1,25 | 1 | 0,2 | 0,3 |
| Glycerin | 5,00 | 3,00 | 10 | 8 | 5 |
| Octylmethoxycinnamat | | | 3 | | 3 |
| Butylmethoxydibenzoylmethan | | | 1 | | 2 |
| Ethylhexyl Triazon | | | | 1 | |
| Glimmer | | 1,00 | | | |
| Eisenoxide | | 1,00 | | | |
| Titandioxid | | 4,50 | | | |
| Vitamin-E-Acetat | | | 0,5 | | 0,5 |
| BHT | | | 0,5 | | |
| Disodium EDTA | | | 0,5 | | 1,0 |
| Parfum | q.s. | q.s. | q.s. | q.s. | q.s. |
| Konservierung | q.s. | q.s. | q.s. | q.s. | q.s. |
| Natriumhydroxid | q.s. | q.s. | q.s. | q.s. | q.s. |
| Farbstoffe usw. | q.s. | q.s. | q.s. | q.s. | q.s. |
| Wasser | ad 100 | ad 100 | ad 100 | ad 100 | ad 100 |
| pH-Wert eingestellt auf | 6.5-7,0 | 6.4-7,5 | 6,0-6,5 | 6,0-7,0 | 6,0-6,5 |
| Volumen % der Grundlage | 85 | 95 | 80 | 97,5 | 90 |
| Volumen % Sauerstoff | 15 | 5 | 20 | 2,5 | 10 |
| | | | | | |
| | 6 | 7 | 8 | 9 | |
| Cetylpalmitat | | 1 | 1,5 | | |

(fortgesetzt)

| | 6 | 7 | 8 | 9 |
|---|---|---|---|---|
| Cetylalcohol | | | | 1 |
| Polysorbate 60 | 1,5 | | 2,5 | |
| Glyceryl Stearate Citrate | | | | |
| Polyglyceryl-3 Methyl Glucose Distea-rate | 3 | | | |
| Ceteareth-20 | | 2 | | |
| Glyceryl Stearate+PEG 30 stearate | | 1,5 | | 2,5 |
| PEG -30 Stearat | | | 2 | 0,7 |
| PEG-40 Stearat | | | | 0,5 |
| Isopropylpalmitate | 5 | | | 2 |
| Cyclomethicon | 5 | | 5 | 2 |
| Squalan | | | 5 | 3 |
| Trilaurin | | 2 | 2 | 2,5 |
| Dimethicon | | | | 3 |
| Dicaprylyl Carbonate | | 1 | | |
| Isohexadecan | 5 | | 2 | 2 |
| Isoeikosan | | 5 | | 2 |
| Mineralöl / Paraffinöl | | | 3 | |
| Hydriertes Polyisobuten | | 5 | | |
| Polydecen | | 5 | | |
| Magnesium-Aluminium-Silikat | 0,3 | 0,4 | 0,15 | 0,25 |
| Polyacrylsäure | | 0,4 | | |
| Alkyl Acrylates Cosspolymere | 0,3 | | | |
| Stärke bzw.-Derivat (Aluminum Starch Octenylsuccinate etc.) | 2 | | | |
| Anorg. Pigmente (wie Boron Nitride, Zeolithe, Kaolin, Silicumoxid, Talkum, Silica Dimethyl Silylate etc.) | 0,5 | 0,7 | 0,25 | 1,5 |
| Citronensäure | | | | 0,1 |
| Glycerin | 10 | 7,5 | 3 | 10 |
| Octylmethoxycinnamat | | 3 | 1 | |
| Butylmethoxydibenzoylmethan | | 2 | 1 | |
| Disodium EDTA | | 0,5 | 0,25 | |
| Parfum | q.s. | q.s. | q.s. | q.s. |
| Konservierung | q.s. | q.s. | q.s. | q.s. |
| Natriumhydroxid | q.s. | q.s. | q.s. | q.s. |
| Farbstoffe usw. | q.s. | q.s. | q.s. | q.s. |
| Wasser | ad 100 | ad 100 | ad 100 | ad 100 |
| pH-Wert eingestellt auf | 5,5-6,0 | 6,0-6,5 | 6,3-6,8 | 5,5-6,0 |
| Volumen % der Grundlage | 87,5 | 92,5 | 90 | 82,5 |
| Volumen % Sauerstoff | 12,5 | 7,5 | 10 | 17,5 |

Vordispergierung des anorganischen Gelbildners und Quellung des Hydrokolloides unter Rühren in der Wasserphase. Vereinigung der auf 75 °C aufgeheizten Fettphase mit der auf 70 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Vakuum und Kühlung. Zugabe der Additive bei 30 °C (Parfüm, Wirkstoffe). Homogenisierung mittels einer Zahnkranzdispergiermaschine (Rotor-Stator-Prinzip) bei 27 °C. Begasung der Emulsion bei 7-8 bar mittels eines dynamischen Schaumgenerators (z.B. von der Firma HANSA INDUSTRIE MIXER) mit Sauerstoff.

**2**

**(schäumbares O/W-Emulsions-Make-up):**

| Emulsion | Gew.% |
|---|---|
| Palmitinsäure | 2,00 |
| Cetylalkohol | 2,00 |
| PEG-100 Stearat [1] | 2,00 |
| Dimethicon [2] | 2,50 |
| Paraffinöl [3] | 9,50 |
| Dicaprylyl Carbonate [4] | 2,00 |
| Glycerin | 3,00 |
| Glimmer | 1,00 |
| Eisenoxid | 1,00 |
| Titandioxid | 4,50 |
| Vitamin A Palmitat | 0,10 |
| Natriumhydroxid | q.s. |
| Konservierung | q.s. |
| Parfum | q.s. |
| Wasser,demineralisiert | ad 100 |
| pH-Wert eingestellt auf 6,0 -7,5 | |
| Zur Herstellung des Schaums werden 85 Vol.-% der Emulsion IV mit 5 Vol.-% Sauerstoff und 5 Vol% Propan/Butan aufgeschäumt[a]. | |
| [1] Myrj 59p, ICI Surfactants, [2] Wacker Silikonöl AK 35, Wacker, [3] Pionier 6301, DEA Mineralöl, [4] Cetiol CC, Henkel Cognis | |

**3**

**(schäumbare OIW-Creme)**

| Emulsion | Gew.-% |
|---|---|
| Stearinsäure | 4,00 |
| Cetylalkohol | 2,00 |
| PEG-30 Stearat [1] | 2,00 |
| Sorbitan Monostearat [2] | 1,50 |
| Paraffinöl [3] | 5,00 |
| Cyclomethicon [4] | 5,00 |
| Vitamin E Acetate | 1,00 |
| Retinylpalmitat | 0,20 |
| Glycerin | 3,00 |
| Alkohol kosmet. | 10,00 |

(fortgesetzt)

| Emulsion | Gew.-% |
|---|---|
| BHT | 0,02 |
| Disodium EDTA | 0,10 |
| Parfüm, Konservierungsmittel, Farbstoffe | q.s. |
| Kaliumhydroxid | q.s. |
| Wasser | ad 100 |
| pH-Wert eingestellt auf 5,0-7,0 | |
| Zur Herstellung des Schaums werden 89 Vol.-% der Emulsion V mit 11 Vol.-% Sauerstoff aufge-schäumt[a]. | |
| [1] Myrj 51, ICI Surfactants, [2] Glycomol S, Akzo, Nobel, [3] Pionier 2076, DEA Mineralöl [4] Dow Corning Fluid 245, Dow Corning | |

**4 (schäumbareO/W-Lotion):**

| Emulsion | Gew.-% |
|---|---|
| Stearinsäure | 4,00 |
| Cetylstearylalkohol | 1,00 |
| PEG-100 Stearat [1] | 1,00 |
| Octyldodecanol | 6,50 |
| Dimethicon [3] | 2,50 |
| Vitamin E Acetat | 2,00 |
| Glycerin | 3,00 |
| Cetyl Ricinoleate | 2,00 |
| Parfüm, Konservierungsmittel, Farbstoffe usw. | q.s. |
| Natriumhydroxid | q.s. |
| Wasser | ad 100 |
| pH-Wert eingestellt auf 6,0-7,5 | |
| Zur Herstellung des Schaums werden 92 Vol.% der Emulsion VI mit 8 Vol.-% eines Treib-gasgemisches aus Propan und Butan (4Vol% ) und Sauerstoff (4 Vol%) aufgeschäumt[a] | |
| [1] Myrj 59p, ICI Surfactants, [3] Wacker Silikonöl AK 50, Wacker | |

**5**

**(schäumbare Sonnenschutz-Cremes):**

| | Emulsion 11 | | Emulsion 12 | |
|---|---|---|---|---|
| | GEW.-% | Vol.-% | Gew.-% | Vol.-%- |
| Stearinsäure | 5,00 | | 1,00. | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglyceride | 4,00 | | 2,00 | |

(fortgesetzt)

| | Emulsion 11 | | Emulsion 12 | |
|---|---|---|---|---|
| | GEW.-% | Vol.-% | Gew.-% | Vol.-%- |
| $C_{12-15}$ Alkyl Benzoat | 10,00 | | 15,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Terephthaliden Dicamphersulfon-säure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| $Na_2H_2EDTA$ | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| **Emulsion 1** | | **85** | | |
| **Emulsion 2** | | | | **90** |
| **Gas (Sauerstoff)** | | **15** | | **10** |
| Zur Herstellung des Schaums werden 85 Vol.-% der Emulsion 12, 13 mit Sauerstoff aufgeschäumt[a]. | | | | |

**6 O/W Emulsionen**

| | 13 | 14 | 15 | 16 | 17 | 18 | 19 |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | 0,75 | 2,00 | | | |
| PEG-40 Stearat | 0,50 | | | | | 2,00 | |
| PEG-100 Stearat | | | 1,50 | | | | |
| Lauryl Methicon Copolyol | | | | 0,75 | | 0,50 | |

(fortgesetzt)

| | 13 | 14 | 15 | 16 | 17 | 18 | 19 |
|---|---|---|---|---|---|---|---|
| Cetyl Phosphat | | | 0,75 | | 1,00 | | |
| Stearyl Alkohol | | | 3,00 | | | 2,00 | 0,50 |
| Cetyl Alkohol | 2,50 | 1,00 | | | 0,50 | | 2,00 |
| UVASorb® K2A | 1,00 | | | 4,00 | | 5,00 | |
| Uvinul® A Plus | 3,00 | 2,50 | 0,50 | 0,25 1,00 | | 0,50 | 4,00 |
| Bis-Ethylhexyloxyphenol Me-thoxyphenyl Triazin | | 1,00 | | | | 1,00 | 0,50 |
| Disodium Phenyl Dibenzimida-zol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | 3,00 | | | | 5,00 |
| Drometrizol Trisiloxan | | | 0,50 | | | 1,00 | |
| Titandioxid T 805 | | 1,50 | | | 1,00 | 0,50 | |
| Titandioxid MT-100Z | 1,00 | | | 3,00 | 1,00 | | |
| $C_{12-15}$ Alkyl Benzoat | | 2,50 | | | | 7,00 | 5,00 |
| Dicaprylylether | | | 3,50 | | 2,00 | | |
| Butylenglycol Dicaprylat/ Di-caprat | 5,00 | | | 5,00 | 3,00 | | |
| Cetearyllsononanoate | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Ethanol | | 2,00 | 1,50 | | 3,00 | 5,00 | 1,00 |

(fortgesetzt)

| | 13 | 14 | 15 | 16 | 17 | 18 | 19 |
|---|---|---|---|---|---|---|---|
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| **Emulsion (Vol%)** | 80 | 85 | 75 | 95 | 90 | 92,5 | 97 |
| **Gas Sauerstoff (Vol%)** | 20 | 15 | 25 | 5 | 10 | 7,5 | 2,5 |

**7**

**Schäume**

| **Emulsion** | 1 | 2 | 3 |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20-Stearat | 3 | | |
| Sorbitanstearat | | 0,8 | |
| $C_{12-15}$ Alkyl Benzoat | 5 | | |
| $C_{12-13}$ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| UVASorb® K2A | | | 2 |
| Uvinul A Plus® | 2 | | |
| NeoHeliopan® AP | | | |
| Phenylbenzimidazol Sulfonsäure | | | |
| Ethylhexylmethoxycinnamat | | | |
| Ethylhexyltriazon | 2 | 2 | 2 |
| Octocrylen | 2 | | |
| Bis-Ethylhexyloxyphenol Methoxyphenyltriazin | | 3 | 3 |
| Glycyrrhiza Inflata | 0,05 | | |
| Kreatin | | | 1 |

| | | | |
|---|---|---|---|
| Kreatinin | | | 0,1 |
| Vitamin E Acetat | | 0,5 | |
| BHT | | | 0,1 |
| $Na_2H_2EDTA$ | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

| **Emulsion** | **4** | **5** | **6** | **7** |
|---|---|---|---|---|
| Stearinsäure | 1,5 | | | |
| Palmitinsäure | | | 3 | 3 |
| Cetylalkohol | | 3 | | |
| Cetylstearylalkohol | | | 2 | 2 |
| Stearylalkohol | 3 | | | |
| PEG-100 Stearat | | 4 | | |
| PEG-40 Stearat | 3 | | | |
| PEG-20-Stearat | | | 3 | 3 |
| Sorbitanstearat | 1 | | | |
| Tridecyl Trimellitate | | 5 | | |
| $C_{12-15}$ Alkyl Benzoat | | | 3 | 3 |
| Butylenglycol Dicaprylat/Dicaprat | 8 | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |

| | | | | |
|---|---|---|---|---|
| Propylenglykol | | | | |
| Glycerin | 5 | | 6 | 6 |
| NeoHeliopan® AP | | 2 | | |
| Phenylbenzimidazol Sulfonsäure | 1 | 4 | 1 | 1 |
| Ethylhexylmethoxycinnamat | 5 | | 4 | 4 |
| Ethylhexyltriazon | | | | |
| Diethylhexylbutamidotriazon | 1 | | | |
| Butyl Methoxydibenzoylmethane | 2,5 | | 2 | 2 |
| Bis-Ethylhexyloxyphenol Methoxyphenyltriazin | 2 | | | |
| Licochalcone A | | | | 0,01 |
| Taurin | | 0,3 | | |
| Vitamin E Acetat | 0,2 | | 0,3 | 0,3 |
| BHT | | 0,05 | | |
| $Na_2H_2EDTA$ | | | 0,4 | 0,4 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Kaliumhydroxid | | | | q.s. |
| Wasser | ad 100,0 | ad 100,0 | ad 100,0 | ad 100,0 |

Zur Herstellung des Schaums werden 80-97 Vol.% der Emulsion I mit 3-20 Vol.-% eines geigneten Gases (z.B. Propan/ Butan, Druckluft, Stickstoff) aufgeschäumt.

**Patentansprüche**

1. Kosmetische Zubereitung in Form einer selbstschäumenden, schaumförmigen, nachschäumenden oder schäumbaren O/W-Emulsion enthaltend 2,5 bis 25 Vol-% molekularen, nicht an Trägerstrukturen gebundenen Sauerstoff - bezogen auf das Gesamtvolumen der Zubereitung - in einem Packmittel bestehend aus einem Bag-on-Valve-System oder Bag-in-Can-System.

2. Zubereitung nach Anspruch 1 **dadurch gekennzeichnet, dass** sich ein Treibmittel zwischen Beutel (Bag) und Aerosolbehälter (Can) befindet, welches umweltfreundlich ist.

3. Zubereitung nach Anspruch 2 **dadurch gekennzeichnet, dass** das Treibmittel ausgewählt wird aus der Gruppe von Stickstoff und Edelgasen.

4. Zubereitung nach Anspruch 1 und/oder 2 **dadurch gekennzeichnet, dass** der Beutel (Bag) mehrlagig mit mindestens einer Sperrschicht ist.

5. Zubereitung nach Anspruch 1 und/oder 2 **dadurch gekennzeichnet, dass** der Aerosolbehälter aus Aluminium, Weißblech oder Kunststoff ist.

6. Zubereitung nach Anspruch 1 **dadurch gekennzeichnet, dass** der Sauerstoffgehalt - bezogen auf das Gesamtvolumen der Zubereitung - aus dem Bereich von 5 bis 20 Vol.-% gewählt wird.

7. Zubereitung nach Anspruch 1 und/oder 6 **dadurch gekennzeichnet, dass** der Sauerstoffgehalt - bezogen auf das Gesamtvolumen der Zubereitung - aus dem Bereich von 7,5 bis 15 Vol.-% gewählt wird.

Europäisches Patentamt
European Patent Office
Office européen des brevets

# EUROPÄISCHER RECHERCHENBERICHT

Nummer der Anmeldung
EP 10 00 9667

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 1 352 641 A (BEIERSDORF AG (DE)) 15. Oktober 2003 (2003-10-15) * Absatz [0001] * * Absatz [0017] * * Anspruch 1; Beispiele 1,2 * ----- | 1-7 | INV. A61Q1/02 A61Q5/00 A61Q17/04 A61Q19/00 A61K8/06 A61K8/22 |
| Y | GB 483 390 A (HEINRICH HERMANN HEER) 20. April 1938 (1938-04-20) * Seite 1, Zeile 69 - Zeile 74 * * Beispiel 1 * ----- | 1-7 | |
| Y | EP 0 379 765 A (VARIX AG (LI)) 1. August 1990 (1990-08-01) * Seite 2, Zeile 53 - Seite 3, Zeile 6 * * Seite 3, Zeile 53 - Seite 4, Zeile 5 * * Seite 4, Zeile 6 - Zeile 8 * * Ansprüche 1,5,12; Beispiele I-V * ----- | 1-7 | |
| Y | US 5 874 093 A (ELIAZ ET AL) 23. Februar 1999 (1999-02-23) * Spalte 1, Zeile 32 - Spalte 2, Zeile 14 * * Spalte 3, Zeile 37 - Zeile 56 * * Spalte 3, Zeile 57 - Zeile 60 * * Ansprüche 1,8,25-32; Beispiele I-VII * ----- | 1-7 | RECHERCHIERTE SACHGEBIETE (IPC) |
| | | | A61Q A61K |
| Y | DE 203 01 831 U1 (CCL RAPID SPRAY GMBH & CO KG [DE]) 17. April 2003 (2003-04-17) * das ganze Dokument * ----- | 1-7 | |
| Y | US 2004/013633 A1 (NOVIKOV ALEXANDER [US] ET AL) 22. Januar 2004 (2004-01-22) * Absatz [0021] * ----- | 1-7 | |

Der vorliegende Recherchenbericht wurde für alle Patentansprüche erstellt

1

| Recherchenort | Abschlußdatum der Recherche | Prüfer |
|---|---|---|
| München | 24. März 2011 | Diebold, Alain |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 10 00 9667

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-03-2011

| Im Recherchenbericht angeführtes Patentdokument | Datum der Veröffentlichung | Mitglied(er) der Patentfamilie | Datum der Veröffentlichung |
|---|---|---|---|
| EP 1352641 A | 15-10-2003 | DE 10216503 A1 | 19-02-2004 |
| GB 483390 A | 20-04-1938 | KEINE | |
| EP 0379765 A | 01-08-1990 | KEINE | |
| US 5874093 A | 23-02-1999 | CA 2221122 A1 | 15-05-1998 |
| DE 20301831 U1 | 17-04-2003 | KEINE | |
| US 2004013633 A1 | 22-01-2004 | AT 347875 T | 15-01-2007 |
| | | AU 2003253832 A1 | 09-02-2004 |
| | | CA 2492002 A1 | 29-01-2004 |
| | | DE 60310419 T2 | 20-09-2007 |
| | | EP 1536758 A1 | 08-06-2005 |
| | | ES 2275120 T3 | 01-06-2007 |
| | | MX PA05000720 A | 08-04-2005 |
| | | WO 2004009047 A1 | 29-01-2004 |
| | | US 2010061955 A1 | 11-03-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 20301831
- EP 972923 A
- EP 1061006 A
- EP 586295 A